# EUROPEAN PATENT APPLICATION

(11) **EP 4 129 362 A1**
(43) Date of publication of application: **08.02.2023**
(21) Application number: 21776362.2
(22) Date of filing: 09.03.2021
(51) Int. Cl.: A61M 5/172, A61M 5/142, G16H 20/17, A61B 5/145

(54) **METHOD AND COMPUTER PROGRAM FOR CONTROLLING INJECTION OF MEDICINAL FLUID**

(30) Priority: 23.03.2020 KR 20200035074
(71) Applicant: Eoflow Co., Ltd., Seongnam-si, Gyeonggi-do 13605 (KR)
(72) Inventor: BIN, In Wook, Yongin-si Gyeonggi-do 16923 (KR); KIM, Ji Hwan, Seoul 07592 (KR)
(74) Representative: Mewburn Ellis LLP
(86) International application number: PCT/KR2021/002893
(87) International publication number: WO 2021/194131

(57) **Abstract**

Provided is a method of controlling liquid medication injection including: receiving, by an electronic device for controlling liquid medication injection, blood glucose values measured by a liquid medication injection device in which a liquid medication injection logic is programmed; generating, by the electronic device for controlling liquid medication injection, a blood glucose change pattern by using the blood glucose values over time; generating, by the electronic device for controlling liquid medication injection, a signal for changing the liquid medication injection logic taking into account a directivity of the change in the blood glucose value according to the blood glucose change pattern; and transmitting, by the electronic device for controlling liquid medication injection, the signal for changing the liquid medication injection logic to the liquid medication injection device.

## Description

### TECHNICAL FIELD

The present disclosure relates to a method of controlling liquid medication injection, and a computer program.

### BACKGROUND ART

In general, an insulin injection device such as an insulin injection device is used to inject a liquid medication into a patient's body. Although such insulin injection devices are sometimes used by professional medical staff such as doctors or nurses, in most cases, it is used by general public such as the patients themselves or guardians. Diabetic patients, especially pediatric diabetic patients, need to inject liquid medications such as insulin into the body at regular intervals. Therefore, an insulin injection device in a patch-form that is used by being attached to human body for a certain period of time is being developed. The above insulin injection device may be used by being attached to the body such as the patient's abdomen or waist in a patch-form for a certain period of time.

The insulin injection device may be designed to inject insulin under the control of an injection amount program designed taking into account the patient's condition. When the insulin is injected as programmed by the insulin injection device, an accidental risk may arise.

Therefore, the insulin injection device needs to inject insulin taking into account a situation in which the patient's blood glucose value is low.

### PRIOR ART DOCUMENT

### PATENT LITERATURE

(Patent Document 1) 0001) Patent Publication No. 2009-0095073

### DETAILED DESCRIPTION OF THE DISCLOSURE

### TECHNICAL PROBLEM

The present disclosure provides a method of controlling liquid medication injection and a computer program.

### TECHNICAL SOLUTION TO PROBLEM

According to one or more embodiments of the present disclosure, a method of controlling liquid medication injection includes: receiving, by an electronic device for controlling liquid medication injection, blood glucose values measured by a liquid medication injection device in which a liquid medication injection logic is programmed; generating, by the electronic device for controlling liquid medication injection, a blood glucose change pattern by using the blood glucose values over time; generating, by the electronic device for controlling liquid medication injection, a signal for changing the liquid medication injection logic taking into account a directivity of change in the blood glucose value according to the blood glucose change pattern; and transmitting, by the electronic device for controlling liquid medication injection, the signal for changing the liquid medication injection logic to the liquid medication injection device.

The generating of the signal for changing the liquid medication injection logic may include generating, by the electronic device for controlling liquid medication injection, the signal for changing the liquid medication injection logic taking into account a future blood glucose value according to the blood glucose change pattern and a directivity of the change.

The signal for changing the liquid medication injection logic may be one of a liquid medication injection stop signal or a liquid medication injection decrease signal.

The method may further include, after the transmitting to the liquid medication injection device, calculating, by the electronic device for controlling liquid medication injection, a current blood glucose value or a future blood glucose value based on a current time point, and determining whether to restart the liquid medication injection logic taking into account the current blood glucose value or the future blood glucose value.

The method may further include, after the transmitting to the liquid medication injection device, when a preset first standby time expires, transmitting, by the electronic device for controlling liquid medication injection, a liquid medication injection logic restart signal or a liquid medication injection logic stop signal to the liquid medication injection device after re-determining whether to restart the liquid medication injection logic taking into account a directivity of the change in the blood glucose value according to the blood glucose change pattern that is calculated based on a current time point.

The method may further include, after the transmitting to the liquid medication injection device, when a preset second standby time expires, automatically transmitting, by the electronic device for controlling liquid medication injection, a liquid medication injection logic restart signal to the liquid medication injection device.

The method may further include: after the transmitting to the liquid medication injection device, receiving, by the electronic device for controlling liquid medication injection, a blood glucose value measured during a set injection stop time period from a time point when a liquid medication injection stop signal is generated; determining, by the electronic device for controlling liquid medication injection, whether the received measured blood glucose value exceeds a normal blood glucose range; and when the measured blood glucose value exceeds the normal blood glucose range, transmitting a signal for restarting the liquid medication injection logic.

According to one or more embodiments of the present disclosure, an electronic device for controlling liquid medication injection, which communicates with a liquid medication injection device in which a liquid medication injection logic is programmed, includes a processor and a communication unit, wherein the processor is configured to execute instructions included in an injection function controller, and the injection function controller receives the measured blood glucose values, generates a blood glucose change pattern by using the blood glucose values over time, generates a signal for changing the liquid medication injection logic taking into account the directivity of change in the blood glucose value according to the blood glucose change pattern, and transmits the signal for changing the liquid medication injection logic to the liquid medication injection device.

### EFFECTS OF THE DISCLOSURE

According to an embodiment of the present disclosure, while insulin is injected according to an injection logic, a liquid medication injection function may be adjusted based on a directivity of change in blood glucose values.

### BRIEF DESCRIPTION OF THE DRAWINGS

FIG. 1 is a block diagram of an injection function controller according to embodiments of the present disclosure.
FIG. 2 is a block diagram of an electronic device for controlling liquid medication injection according to embodiments of the present disclosure.
FIGS. 3 to 5 are flowcharts for describing a method of controlling liquid medication injection according to embodiments of the present disclosure.
FIG. 6 is a diagram illustrating an injection time period, an injection stop time period, an injection restart time period, etc. of liquid medication injection.
FIGS. 7 to 9 are diagrams illustrating a liquid medication injection system according to embodiments of the present disclosure.

### BEST MODE

Provided is a method of controlling liquid medication injection including: receiving, by an electronic device for controlling liquid medication injection, blood glucose values measured by a liquid medication injection device in which a liquid medication injection logic is programmed; generating, by the electronic device for controlling liquid medication injection, a blood glucose change pattern by using the blood glucose values over time; generating, by the electronic device for controlling liquid medication injection, a signal for changing the liquid medication injection logic taking into account a directivity of the change in the blood glucose value according to the blood glucose change pattern; and transmitting, by the electronic device for controlling liquid medication injection, the signal for changing the liquid medication injection logic to the liquid medication injection device.

### MODE OF THE DISCLOSURE

As the present disclosure allows for various changes and numerous embodiments, particular embodiments will be illustrated in the drawings and described in detail in the written description. The attached drawings for illustrating one or more embodiments are referred to in order to gain a sufficient understanding, the merits thereof, and the objectives accomplished by the implementation. However, the embodiments may have different forms and should not be construed as being limited to the descriptions set forth herein.

In the present specification, the expression "include" or "may include" refers to existence of a corresponding function, operation, or element, and does not limit one or more additional functions, operations, or elements. In the present specification, it is to be understood that the terms such as "including," "having," and "comprising" are intended to indicate the existence of the features, numbers, steps, actions, components, parts, or combinations thereof disclosed in the specification, and are not intended to preclude the possibility that one or more other features, numbers, steps, actions, components, parts, or combinations thereof may exist or may be added.

In the present disclosure, the expression "or" includes any or all combinations of words enumerated together. For example, the expression "A or B" may include A, may include B, or may include both A and B.

In the present disclosure, expressions including ordinal numbers, such as "first" and "second," etc., may modify various elements. However, such elements are not limited by the above expressions. For example, the above expressions do not limit the sequence and/or importance of the elements. The above expressions are used merely for the purpose of distinguishing an element from the other elements. For example, a first user device and a second user device indicate different user devices although both of them are user devices. For example, a first element could be termed a second element, and similarly, a second element could be also termed a first element without departing from the scope of the present disclosure.

In the case where an element is referred to as being "connected" or "accessed" to other elements, it should be understood that not only the element is directly connected or accessed to the other elements, but also another element may exist between them. Contrarily, when an element is referred to as being "directly coupled" or "directly connected" to any other element, it should be understood that no element is interposed therebetween.

In addition, the terms such as "module", "... unit", "part", etc. provided herein indicates a unit performing at least one function or operation, and may be realized by hardware, software, or a combination of hardware and software. In addition, a plurality of "modules", "units", "parts", etc. may be integrated into at least one module or chip and may be implemented as at least one processor, except when each needs to be implemented in individual specific hardware.

The terms used in the disclosure are only used to describe specific embodiments, and are not intended to limit the disclosure. The singular expression includes the plural expression unless the context clearly dictates otherwise.

Unless defined otherwise, all terms used herein, including technical and scientific terms, have the same meaning as commonly understood by those of skill in the art to which the disclosure pertains.

Such terms as those defined in a generally used dictionary are to be interpreted to have the meanings equal to the contextual meanings in the relevant field of art, and are not to be interpreted to have ideal or excessively formal meanings unless clearly defined in the disclosure.

Hereinafter, one or more embodiments of the disclosure will be described in detail with reference to accompanying drawings.

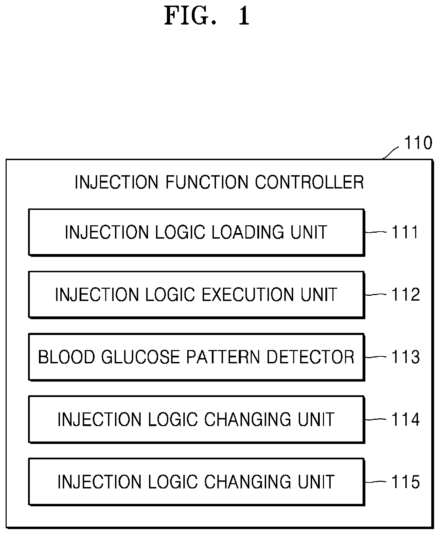

FIG. 1 is a block diagram of an injection function controller 110 according to embodiments of the present disclosure.

The injection function controller 110 may include an injection logic loading unit 111, an injection logic execution unit 112, a blood glucose pattern detector 113, an injection logic changing unit 114, and an injection logic reactivation unit 115.

The injection logic loading unit 111 may load an injection logic to be executed. The injection logic may be received from a server of a medical institution in which the treatment of a user has been carried out. Alternatively, the injection logic may be set by the user. Loading may refer to executing of the injection logic after storing the injection logic in a temporary storage memory.

The injection logic execution unit 112 may execute the loaded injection logic. The injection logic may refer to a program for injecting a liquid medication of an injection dose at the time of injection.

The blood glucose pattern detector 113 may perform a function of detecting a change pattern of a blood glucose value in the process of performing insulin injection by the injection logic. More specifically, the blood glucose pattern detector 113 may receive measured blood glucose values from the liquid medication injection device or a remote blood glucose measuring device, and calculate a blood glucose change pattern based on the measured blood glucose values. Here, the blood glucose change pattern represents a change in blood glucose values over time. When a first blood glucose value and a second blood glucose value are measured, the blood glucose change pattern may include directivity of the first blood glucose value and the second blood glucose value with respect to time, an inflection point where the blood glucose value rises and falls, an inflection point where the blood glucose value falls and rises, a difference value from a reference blood glucose value, etc.

The blood glucose pattern detector 113 may determine whether the blood glucose values have a rising directivity or a falling directivity based on the measured blood glucose values. The blood glucose pattern detector 113 may determine the changing directivity of blood glucose value over time through the blood glucose change pattern generated based on the measured blood glucose values. The blood glucose pattern detector 113 may predict one or more future blood glucose values by using the blood glucose change pattern and the past blood glucose change pattern. The blood glucose pattern detector 113 may predict future blood glucose values after a first blood glucose change pattern, by using the first glucose change pattern determined based on the blood glucose values of a first user and a second blood glucose change pattern of the first user in the past. The blood glucose pattern detector 113 may predict future blood glucose value after the first blood glucose change pattern of the first user by using the first blood glucose change pattern determined based on the blood glucose values of the first user and a third blood glucose change pattern of a second user. Here, the second blood glucose change pattern or the third blood glucose change pattern may include one or more blood glucose change patterns. The second user may include one or more users.

The blood glucose pattern detector 113 may calculate a directivity of the future blood glucose values corresponding to the measured blood glucose values and future blood glucose values by using a blood glucose change determination algorithm. In detail, based on a blood glucose value at a first time point and a blood glucose value at a second time point, a blood glucose value at a third time point in the future after the first and second time points may be calculated by using the blood glucose change pattern that is accumulated data about change in the past blood glucose values of each user.

The blood glucose pattern detector 113 may search the blood glucose values at time points corresponding to the first to second time points for measurement data having the same blood glucose change pattern, and may determine the blood glucose value at the third time point in the future by using the blood glucose change pattern that is determined based on the found measurement data. The measurement data having the same blood glucose change pattern may be selected from the past data of the corresponding user or past data of another user.

The blood glucose pattern detector 113 may predict the blood glucose change pattern of the user in further consideration of the future blood glucose values.

The blood glucose pattern detector 113 may determine a corresponding blood glucose change pattern taking into further account of liquid medication injection information, in addition to the blood glucose change pattern made by the measured blood glucose values.

In another embodiment, the blood glucose pattern detector 113 may determine whether the measured blood glucose value exceeds a normal blood glucose range. The blood glucose pattern detector 113 may determine whether the predicted future blood glucose value exceeds the normal blood glucose range. When it is determined that the future blood glucose value exceeds the normal blood glucose range, the blood glucose pattern detector 113 may generate a signal for generating a liquid medication injection signal. The generated signal for generating the liquid medication injection signal may be generated prior to the corresponding future time point.

The blood glucose pattern detector 113 may detect whether the measured blood glucose value is equal to or less than a preset minimum blood glucose value, detect the directivity of the obtained blood glucose values based on the measured blood glucose values, or detect whether the future blood glucose value obtained based on the measured blood glucose values is equal to or less than the minimum blood glucose value.

When it is detected that the blood glucose pattern received by the blood glucose pattern detector 113 satisfies a certain condition, the injection logic changing unit 114 performs a function of changing an injection logic. As such, the liquid medication may be injected with a logic other than the injection logic stored in correspondence with the user. For example, the injection logic changing unit 114 may stop the liquid medication injection or change an injection amount or an injecting timing.

When a first signal for changing the liquid medication injection is generated and then a second signal for changing the liquid medication injection is generated again, the injection logic changing unit 114 generates the second signal after a preset first standby time passes from the first signal. Here, the first standby time may be set differently from a kind of the first signal. As such, it may be possible to prevent frequent generation of signals for changing the liquid medication injection. The injection logic changing unit 114 may generate a liquid medication injection stop signal or a liquid medication injection restart signal taking into account the directivity in the blood glucose change and future blood glucose values through the blood glucose change pattern. For example, when the future blood glucose value is equal to or less than the preset minimum blood glucose value and the directivity of the change in the blood glucose value is decreasing, the injection logic changing unit 114 may transmit a signal for stopping the insulin injection to the liquid medication injection device. The injection logic changing unit 114 may transmit an injection stop signal for stopping the insulin injection to the liquid medication injection device. Here, the minimum blood glucose value may be determined to be equal to or greater than a general reference blood glucose value, as an average blood glucose value of the user, or based on, for example, height, weight, date of birth, and blood type, that is, body information of the user related to the average blood glucose value of the user.

When the future blood glucose value is equal to or greater than a preset start blood glucose value and the directivity of the change in the blood glucose value is increasing, the injection logic changing unit 114 may transmit a signal for restarting insulin injection to the liquid medication injection device. The injection logic changing unit 114 may transmit an injection stop signal for stopping the insulin injection to the liquid medication injection device.

In another embodiment, the injection logic changing unit 114 may transmit the liquid medication injection stop signal to the liquid medication injection device through an injection controller.

After the generation of the liquid medication injection stop signal, the injection logic changing unit 114 may automatically generate a liquid medication injection restart signal when a second standby time elapses and an injection logic reactivation unit 115 may be executed. The second standby time may be set to be longer than the first standby time. As such, it may be possible to prevent a health problem from occurring by stopping the liquid medication injection. The injection logic reactivation unit 115 may perform a function of reactivating the injection logic stored in advance, based on the blood glucose change pattern. Here, the blood glucose change pattern may include a directivity of future blood glucose values, a directivity of the change in the blood glucose value, etc. When it is determined that the future blood glucose value predicted based on the measured blood glucose values is equal to or greater than a reference blood glucose value, the injection logic reactivation unit 115 may transmit a signal for reactivating the injection logic to the liquid medication injection device.

As such, the electronic device for controlling the liquid medication injection may implement operations of stopping and restarting the liquid medication such as insulin, and thus, occurrence of unexpected risk of hypoglycemia or hyperglycemia may be prevented.

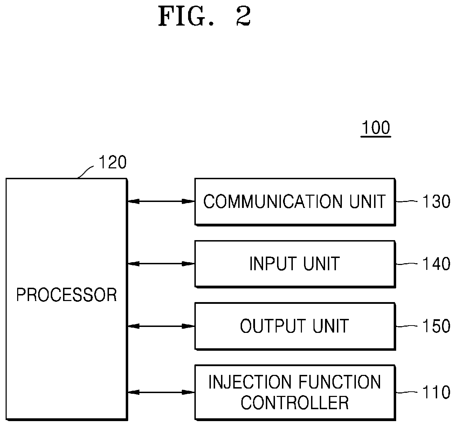

FIG. 2 is a block diagram of an electronic device 100 for controlling liquid medication injection according to embodiments of the present disclosure.

A processor 120 is a configuration for overall controlling the electronic device 100 for controlling liquid medication injection. In particular, the processor 120 controls overall operations of the electronic device 100 for controlling the liquid medication injection by using various programs stored in the injection function controller 110 of the electronic device 100 for controlling the liquid medication injection. For example, the processor 120 may include a central processing unit (CPU), random access memory (RAM), read-only memory (ROM), and a system bus. Here, the ROM is an element in which a instruction set for system booting is stored, and the CPU copies an operating system (O/S) stored in a memory of the electronic device 100 for controlling the liquid medication injection to the RAM and executes the O/S to boot the system. When booting is completed, the CPU may copy various applications stored in the injection function controller 110 to the RAM and executes the various applications to perform various operations. In the above description, the electronic device 100 for controlling the liquid medication injection includes only one CPU, but the electronic device 100 for controlling the liquid medication injection may be implemented to include a plurality of CPUs (or digital signal processor (DSP), system on chip (SoC), etc.).

According to an embodiment of the present disclosure, the processor 120 may be implemented as a DSP, a microprocessor, or a time controller (TCON) for processing a digital signal. However, one or more embodiments are not limited thereto, and the processor 120 may include a CPU, a micro controller unit (MCU), a micro processing unit (MPU), a controller, an application processor (AP), or one or more of a communication processor (CP) and an ARM processor, or may be defined by a corresponding term. In addition, the processor 120 may be implemented as an SoC or large-scale integration (LSI) having a built-in processing algorithm, or may be implemented as a field programmable gate array (FPGA) type.

The electronic device 100 for controlling the liquid medication injection may further include a storage medium (not shown) that stores various data for overall operations, such as a program for processing or controlling the processor 120. The storage medium may store a plurality of application programs (or applications) driven in the electronic device 100 for controlling the liquid medication injection, data and instructions for operating the electronic device 100 for controlling the liquid medication injection. At least some of the application programs may be downloaded from an external server via wireless communication. In addition, at least some of the application programs may exist on the electronic device 100 for controlling the liquid medication injection from the time of release for basic functions of the electronic device 100 for controlling the liquid medication injection. The application program may be stored in the storage medium, and may be driven by the processor 120 to perform an operation (or function) of the electronic device 100 for controlling the liquid medication injection.

The communication unit 130 is configured to transmit and receive data to and from devices such as a server and other user terminals. The communication unit 130 may include a Bluetooth communication unit, a BLE (Bluetooth Low Energy) communication unit, a near field communication unit, a WLAN (Wi-Fi) communication unit, a Zigbee communication unit, an infrared (IrDA, infrared Data Association) communication unit, a WFD (Wi-Fi Direct) communication unit, an ultra wideband (UWB) communication unit, a short-distance communication unit such as an Ant+ communication unit, and a mobile communication network.

An input unit 140 may include a user interface for inputting various types of information to the electronic device 100 for controlling the liquid medication injection.

The electronic device 100 for controlling the liquid medication injection may display, on an output unit 150, a liquid medication injection logic, a liquid medication injection status, a blood glucose value status, an effect, etc. generated by the injection function controller 110. According to an embodiment of the present disclosure, the output unit 150 may display a user interface according to a user input that is input through the input unit 140. The output unit 150 may output stored graphic data, visual data, auditory data, and vibration data under the control of the injection function controller 110.

The output unit 150 may be implemented as various types of display panel. For example, the display panel may be implemented via various display techniques such as liquid crystal display (LCD), organic light emitting diode (OLED), active-matrix organic light-emitting diode (AM-OLED), liquid crystal on silicon (LcoS), digital light processing (DLP), etc. Also, the output unit 150 may be coupled to at least one of a front area, a side area, and a rear area of the display panel in the form of a flexible display.

The output unit 150 may be implemented as a touch screen having a layered-structure. In addition to the display function, the touch screen may have a function of detecting not only a touch input position and a touched area, but also a touch input pressure, and may also have a function of detecting proximity touch, as well as a real-touch.

The electronic device 100 for controlling the liquid medication injection may include the injection function controller 110 and the communication unit 130 therein. The electronic device 100 for controlling the liquid medication injection may not include at least one from the processor 120, the input unit 140, and the output unit 150, and may be electrically connected to at least one from the processor 120, the input unit 140, and the output unit 150 provided in a remote device.

The electronic device 100 for controlling the liquid medication injection may be an electronic device that includes the injection function controller 110 and the control unit 130. The electronic device 100 for controlling the liquid medication injection may be connected to a remote liquid medication injection device via the communication unit 130 and may be operated. The electronic device 100 for controlling the liquid medication injection may receive the measured blood glucose values from the liquid medication injection device or may measure the blood glucose value by including an additional blood glucose measuring device. The electronic device 100 for controlling the liquid medication injection may be electrically connected to the blood glucose measuring device or may be connected to the blood glucose measuring device via a communication network.

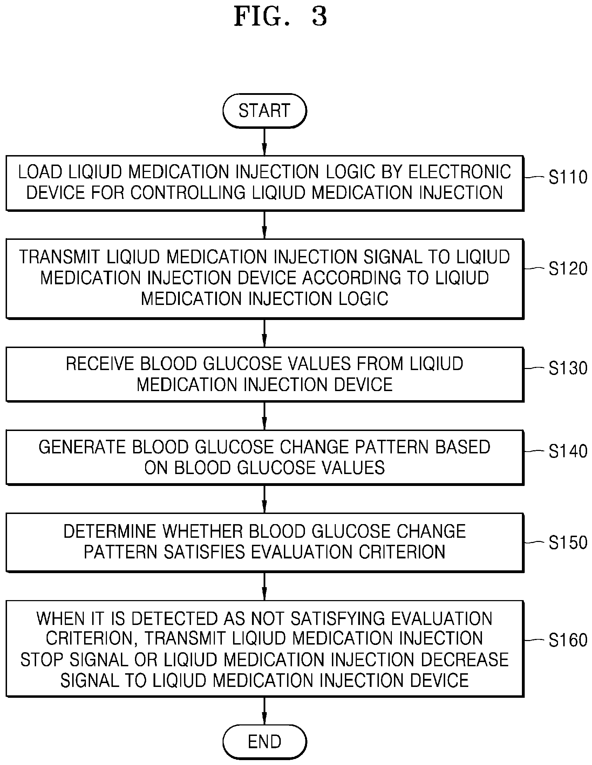

FIGS. 3 to 5 are flowcharts for describing a method of controlling liquid medication injection according to embodiments of the present disclosure.

As shown in FIG. 3, in S110, the electronic device 100 for controlling the liquid medication injection loads the liquid medication injection logic in a volatile memory.

In S120, the electronic device 100 for controlling the liquid medication injection may transmit a liquid medication injection signal to the liquid medication injection device by the liquid medication injection logic.

In S130, the electronic device 100 for controlling the liquid medication injection may receive the blood glucose value from the liquid medication injection device, but is not limited thereto, and may receive the blood glucose value from the blood glucose measuring device. The electronic device 100 for controlling the liquid medication injection may receive an input of the blood glucose value through a user input.

In S140, the electronic device 100 for controlling the liquid medication injection may generate a blood glucose change pattern based on the measured blood glucose values. Here, the blood glucose change pattern represents a change in blood glucose values over time. When a first blood glucose value and a second blood glucose value are measured, the blood glucose change pattern may include directivity of the first blood glucose value and the second blood glucose value with respect to time, an inflection point where the blood glucose value rises and falls, an inflection point where the blood glucose value falls and rises, a difference value from a reference blood glucose value, etc.

The electronic device 100 for controlling the liquid medication injection may calculate a directivity of the future blood glucose values corresponding to the measured blood glucose values and future blood glucose values by using a blood glucose change determination algorithm. The electronic device 100 for controlling the liquid medication injection may search the blood glucose values at time points corresponding to the first to second time points for measurement data having the same blood glucose change pattern, and may determine the blood glucose value at a third time point in the future by using the blood glucose change pattern that is determined based on the found measurement data. The measurement data having the same blood glucose change pattern may be selected from the past data of the corresponding user or past data of another user. The electronic device 100 for controlling the liquid medication injection may predict the blood glucose change pattern of the user taking into further account of the future blood glucose values. The electronic device 100 for controlling the liquid medication injection may determine a corresponding blood glucose change pattern taking into further account of liquid medication injection information, in addition to the blood glucose change pattern made by the measured blood glucose values.

In S150, the electronic device 100 for controlling the liquid medication injection may determine whether the blood glucose change pattern satisfies an evaluation criterion. Here, the evaluation criterion is a criterion for evaluating whether the blood glucose change pattern is normal, and may be set based on a current blood glucose value, a future blood glucose value, a directivity of change in the blood glucose value, etc. The evaluation criterion may be adjusted according to the average blood glucose value of each user by using the measured blood glucose values.

In another embodiment, the evaluation criteria may be set differently taking into account the average blood glucose value of the user. The evaluation criterion may be set taking into account average blood glucose values of a user group including the user. The user group is determined based on other information related to the user. For example, the user group may include users having at least one similar index from among height, weight, date of birth, blood type, gender, age, and occupation. Here, the index for determining the user group refers to an index having high relevance to the blood glucose change pattern.

For example, a user group is searched for based on height, weight, date of birth, blood type, gender, age, occupation, etc. of a user A, and a minimum blood glucose value that serves as a reference for stopping the liquid medication injection for the user A, based on the average blood glucose value, the minimum blood glucose value, the maximum blood glucose value, etc. of the users included in the user group.

In S160, when it is determined that the blood glucose change pattern of the user does not satisfy the evaluation criterion while the injection is performed by the injection logic based on the measured blood glucose value, the electronic device 100 for controlling the liquid medication injection may change the liquid medication injection logic by transmitting a liquid medication injection stop signal or a liquid medication injection decrease signal to the liquid medication injection device. Here, the liquid medication injection stop signal may be a signal for stopping the liquid medication injection for a predetermined time period, and the liquid medication injection decrease signal may be a signal for decreasing the liquid medication injection dose. The liquid medication injection stop signal or the liquid medication injection decrease signal may be transmitted in the preset first standby time, e.g., 30 minutes or 10 minutes, after the previous transmission of the liquid medication injection change signal. In another embodiment, when a preset second standby time passes after the liquid medication injection stop signal or the liquid medication injection decrease signal is transmitted, the liquid medication injection restart signal may be automatically transmitted.

The electronic device 100 for controlling the liquid medication injection may determine whether a subsequent blood glucose change pattern that is determined by re-considering the blood glucose value measured during the standby time satisfies the evaluation criterion before transmitting a signal regarding the liquid medication injection.

The electronic device 100 for controlling the liquid medication injection may generate a signal for changing the liquid medication injection logic taking into account the blood glucose change pattern and the subsequent blood glucose change pattern. When both the blood glucose change pattern and the subsequent blood glucose change pattern satisfy the evaluation criteria, the liquid medication injection stop signal (or liquid medication injection decrease signal) may not be transmitted. When it is determined that the blood glucose change pattern or the subsequent blood glucose change pattern does not satisfy the evaluation criterion after the standby time, the liquid medication injection stop signal or the liquid medication injection decrease signal may be transmitted.

In another embodiment, when the number of times that the blood glucose change pattern is detected as not satisfying the evaluation criterion is equal to or greater than a certain minimum number of times, the electronic device 100 for controlling the liquid medication injection may transmit the liquid medication injection stop signal or the liquid medication injection decrease signal.

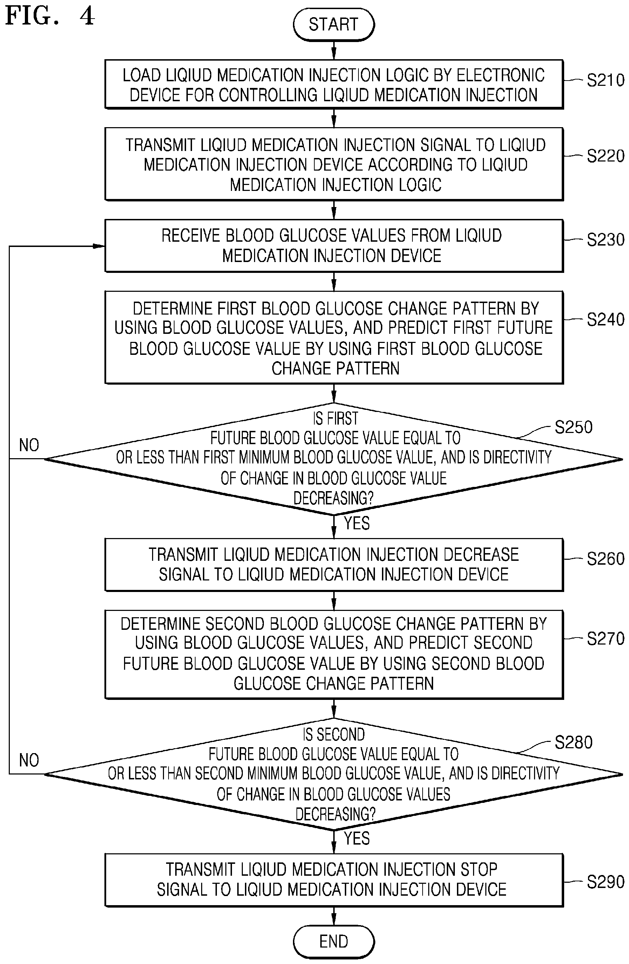

As shown in FIG. 4, in S210, the electronic device 100 for controlling the liquid medication injection may load the liquid medication injection logic. The electronic device 100 for controlling the liquid medication injection may load and execute the liquid medication injection logic in a temporary storage memory, wherein the liquid medication injection logic is received by a request signal. The request signal may be generated by instructions recorded in the electronic device 100 for controlling the liquid medication injection. Alternatively, the request signal may be generated by a user input.

In S220, the electronic device 100 for controlling the liquid medication injection may transmit a liquid medication injection signal to the liquid medication injection device by the liquid medication injection logic.

In S230, the electronic device 100 for controlling the liquid medication injection may receive the blood glucose value from the liquid medication injection device, but is not limited thereto, and may receive the blood glucose values from the blood glucose measuring device. The electronic device 100 for controlling the liquid medication injection may receive an input of the blood glucose value through an input. The electronic device 100 for controlling the liquid medication injection may receive the blood glucose values at a preset time interval.

In S240, the electronic device 100 for controlling the liquid medication injection may determine the first blood glucose change pattern by using the blood glucose values and predict a first future blood glucose value by using the first blood glucose change pattern.

In S250, the electronic device 100 for controlling the liquid medication injection determines whether the first future blood glucose value is equal to or less than a first minimum blood glucose value and the directivity of the change in the blood glucose value is decreasing. The liquid medication injection decrease signal may be implemented to inject only a part, e.g., 50%, of the injection amount included in the liquid medication injection logic. In S260, the electronic device 100 for controlling the liquid medication injection transmits the liquid medication injection decrease signal to the liquid medication injection device.

In S270, the electronic device 100 for controlling the liquid medication injection may determine the second blood glucose change pattern based on the blood glucose values and predict a second future blood glucose value by using the second blood glucose change pattern. In S280 and S290, when the second future blood glucose value is equal to or less than the second minimum blood glucose value and the directivity of the change in the blood glucose value is decreasing, the electronic device 100 for controlling the liquid medication injection may transmit the liquid medication injection stop signal to the liquid medication injection device.

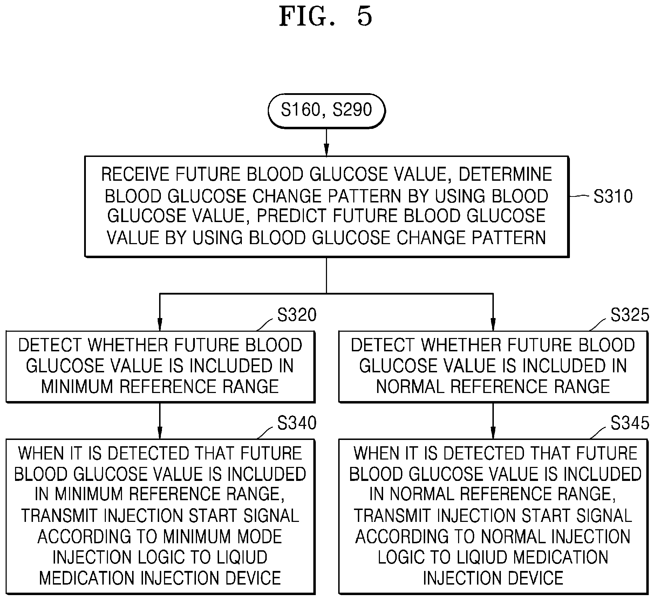

As shown in FIG. 5, after S160, the electronic device 100 for controlling the liquid medication injection may receive the blood glucose value of the user after the liquid medication injection logic is changed in S310. The electronic device 100 for controlling the liquid medication injection determines the blood glucose change pattern by using the blood glucose value and predicts the future blood glucose value by using the blood glucose change pattern.

In S320, the electronic device 100 for controlling the liquid medication injection may detect whether the future blood glucose value is included in a minimum reference range.

In S340, when it is detected that the future blood glucose value of the user is included in the reference range, the electronic device 100 for controlling the liquid medication injection may transmit an injection start signal to the liquid medication injection device according to a minimum mode injection logic.

After S310, the electronic device 100 for controlling the liquid medication injection may detect whether the future blood glucose value is included in the normal reference range.

In S345, when it is detected that the future blood glucose value is included in the normal reference range, the electronic device 100 for controlling the liquid medication injection may transmit an injection start signal to the liquid medication injection device according to a normal injection logic. After there is a change such as stopping or decreasing the liquid medication injection logic, restarting of the liquid medication injection may be processed in two routes. The two routes may be determined based on future blood glucose values.

In another embodiment, the electronic device 100 for controlling the liquid medication injection may execute a liquid medication injection control method in an order of S310, S320, S330, S325, and S345. That is, when the future blood glucose value is equal to or greater than the first minimum reference value and the directivity of the change in the blood glucose value is increasing, the electronic device 100 for controlling the liquid medication injection injects the liquid medication in the minimum mode injection logic, and when the future blood glucose value predicted after a certain standby time passes is equal to or greater than the second minimum reference value and the directivity of the change in the blood glucose value is increasing, the electronic device 100 for controlling the liquid medication injection may inject the liquid medication in the normal mode injection logic.

As such, when it is determined that a risk of blood glucose, e.g., excessive increase or decrease in the blood glucose value, occurs due to the injection according to the insulin injection logic, the electronic device 100 for controlling the liquid medication injection may change the injection of insulin that is a programmed liquid medication.

The electronic device 100 for controlling the liquid medication injection may inject the liquid medication by using the blood glucose value of the user, the future blood glucose value, and the directivity of the change in the blood glucose. In another embodiment, the electronic device 100 for controlling the liquid medication injection may change the programmed function of the liquid medication injection taking into account blood glucose values measured while the liquid medication is injected, the future blood glucose value, and the directivity of the change in the blood glucose.

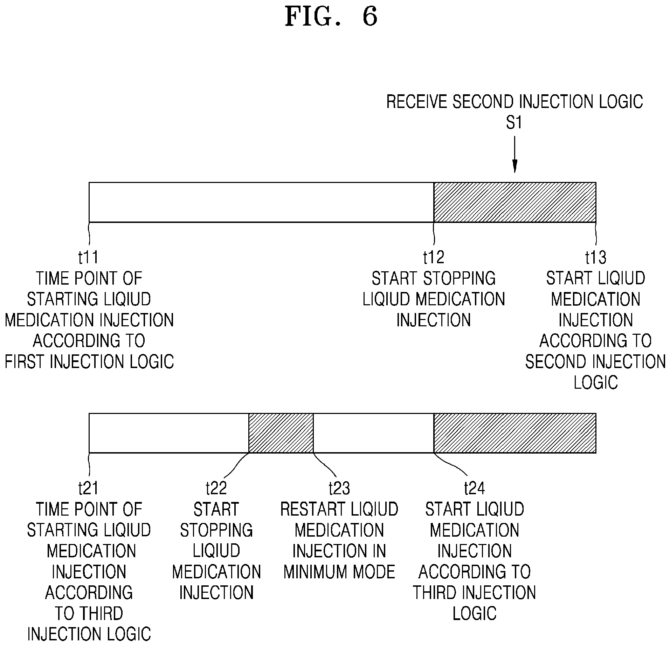

FIG. 6 is a diagram illustrating an injection time period, an injection stop time period, an injection re-start time period, etc. of liquid medication injection.

The electronic device 100 for controlling the liquid medication injection may start the liquid medication injection according to a first injection logic at a time point t11. The electronic device 100 for controlling the liquid medication injection receives the user's blood glucose value measured while the liquid medication injection is performed and monitors the blood glucose values. When it is determined that the blood glucose value is equal to or less than the reference blood glucose value, the electronic device 100 for controlling the liquid medication injection generates a signal for stopping the liquid medication injection from a time point t12. After receiving a second injection logic in which a liquid medication injection amount and a liquid medication injection timing are adjusted (S1), the liquid medication injection according to the second injection logic may be started after a time point t13. The electronic device 100 for controlling the liquid medication injection may prevent a risk of hypoglycemia that suddenly occurs during performing the liquid medication injection logic.

The electronic device 100 for controlling the liquid medication injection may start the liquid medication injection according to a third injection logic at a time point t21. After injecting the liquid medication, the electronic device 100 for controlling the liquid medication injection receives the measured blood glucose value, determines whether a blood glucose value and a change pattern of the blood glucose value satisfy the evaluation criterion. When the blood glucose value and the change pattern of the blood glucose value do not satisfy the evaluation criterion (t22), the electronic device 100 for controlling the liquid medication injection may transmit a signal for stopping the liquid medication injection to the liquid medication injection device.

After stopping the liquid medication injection, the electronic device 100 for controlling the liquid medication injection detects the blood glucose value and the change pattern of the blood glucose value, and when it is determined that the change pattern of the blood glucose value maintains in the minimum reference range, the electronic device 100 for controlling the liquid medication injection may restart the liquid medication injection in the minimum mode from a time point t23. A restart signal may be transmitted to the liquid medication injection device. The blood glucose value of the user is measured while performing the liquid medication injection in the minimum mode and it may be determined whether the blood glucose value of the user is maintained to be equal to or greater than the minimum blood glucose value and to be equal to or less than the maximum blood glucose value.

When it is determined that the blood glucose value of the user is in the range from the minimum blood glucose value to the maximum blood glucose value as a determination result, the electronic device 100 for controlling the liquid medication injection may transmit a signal for starting the liquid medication injection according to the third injection logic to the liquid medication injection device at a time point t24.

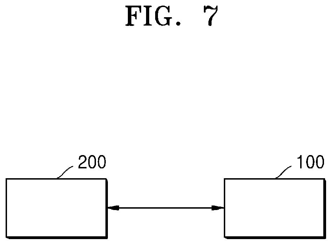

FIGS. 7 to 9 are diagrams illustrating a liquid medication injection system according to embodiments of the present disclosure.

As shown in FIG. 7, a liquid medication injection system may include the electronic device 100 for controlling the liquid medication injection and a liquid medication injection device 200.

The liquid medication injection device 200 may inject liquid medications such as insulin, glucagon, anesthetic, analgesic, dopamine, growth hormone, and smoking cessation aid that has to be injected to the user. The liquid medication injection device 200 may be programmed with a liquid medication injection logic from the electronic device 100 for controlling the liquid medication injection. The liquid medication injection device 200 may inject the liquid medication according to the liquid medication injection signal from the electronic device 100 for controlling the liquid medication injection.

The liquid medication injection device 200 is a device for injecting a liquid medication into the user's body by penetrating through the user's skin, and may further include a unit for storing a material such as a liquid medication that has to be periodically injected to the user, and a unit for controlling the liquid medication to be injected with a preset injection amount. The liquid medication injection device 200 may be controlled so that an injection amount that has to be injected is injected according to the injection signal from the electronic device 100 for controlling the liquid medication injection or the liquid medication injection logic by using the unit for storing the material, the unit for injection, etc., but is not limited thereto, and a signal generated by the electronic device 100 for controlling the liquid medication injection may be transmitted through an additional injection controller.

Information such as the measured blood glucose value (the blood glucose value measured before injection, the blood glucose value measured after the injection, the measured blood glucose value measured in a preset time unit) and the actual injection amount may be transmitted to the electronic device 100 for controlling the liquid medication injection.

Additionally, the liquid medication injection device 200 may transmit a device status message, a biometric value measurement message, a liquid medication injection message, etc. to the electronic device 100 for controlling the liquid medication injection. For example, the liquid medication injection device 200 may transmit the device status message including information on a remaining battery capacity of the device, whether the device is booted successfully, whether the injection is successful, etc. to a controller. Messages transmitted to the electronic device 100 for controlling the liquid medication injection may be transmitted to a user terminal through the injection controller.

The liquid medication injection device 200 may also be implemented to communicate only with the electronic device 100 for controlling the liquid medication injection that has been approved through certain procedure. In addition, the liquid medication injection device 200 may include an injection device related to injection. The liquid medication injection device 200 may be implemented to include the injection function controller 110 shown in FIG. 1. The liquid medication injection device 200 may be electrically connected to the injection function controller 110. The liquid medication injection device 200 may execute operations such as liquid medication injection, stopping of the liquid medication injection, restart of the liquid medication injection, etc. via the injection function controller 110.

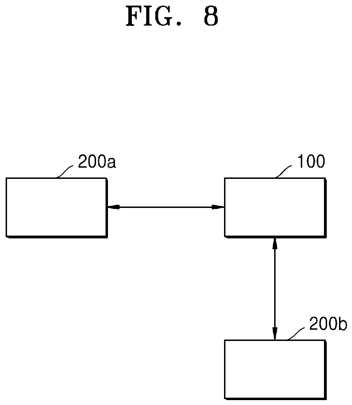

The liquid medication injection system may be implemented by communicating with the electronic device 100 for controlling the liquid medication injection, a liquid medication injection device 200a, and a biometric value measuring device 200b. As shown in FIG. 8, the liquid medication injection device 200a and the biometric value measuring device 200b may be implemented separately.

The electronic device 100 for controlling the liquid medication injection may transmit an injection-related signal to the liquid medication injection device 200a, and the biometric value measuring device 200b may transmit a biometric value measurement signal to the electronic device 100 for controlling the liquid medication injection.

The electronic device 100 for controlling the liquid medication injection may receive the biometric value measurement signal while the liquid medication is injected or not injected. The electronic device 100 for controlling the liquid medication injection may detect a change pattern in the biometric value such as the blood glucose value of the user.

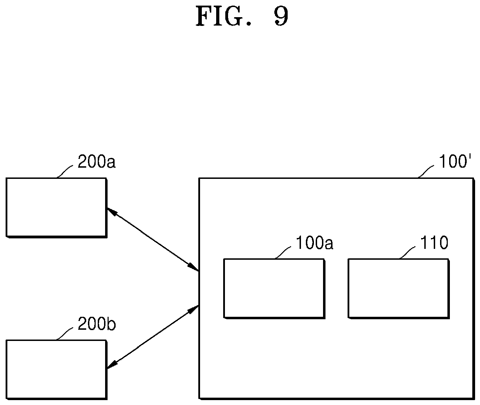

As shown in FIG. 9, an electronic device 100' for controlling the liquid medication injection may be implemented to include an injection controller 100a and the injection function controller 110.

The injection controller 100a performs a function of transmitting/receiving data to/from the liquid medication injection device 200a, transmits a control signal related to the injection of a liquid medication such as insulin to the liquid medication injection device 200a, and may receive a control signal related to the measurement of the biometric value such as the blood glucose from the liquid medication injection device 200a. The control signals related to the measured biometric values and the liquid medication injection may include a plurality of operations with respect to a certain time period.

The injection controller 100a may transmit an instruction request to measure the current state of the user to the liquid medication injection device 200a, and may receive measurement data from the biometric value measuring device 200b in response to the instruction request.

The above apparatus described herein may be implemented using hardware components, software components, and/or combination of the hardware components and the software components. For example, the apparatuses and the components described in the embodiments may be implemented using, for example, a processor, a controller, an arithmetic logic unit (ALU), a digital signal processor, a microcomputer, a field programmable gate array (FPGA), a programmable logic unit (PLU), a microprocessor, or one or more general-purpose computers or specific-purpose computers such as any other device capable of responding to and executing instructions in a defined manner. The processing device may run an operating system (OS) and one or more software applications that run on the OS. The processing device also may access, store, manipulate, process, and create data in response to execution of the software. For convenience of comprehension, the description of a processing device is used as singular; however, one of ordinary skill in the art will be appreciated that a processing device may include multiple processing elements and/or multiple types of processing elements. For example, a processing device may include multiple processors or a processor and a controller. In addition, different processing configurations are possible, such as a parallel processors.

The software may include a computer program, a code, an instruction, or a combination of one or more thereof, for independently or collectively instructing or configuring the processing device to operate as desired. Software and/or data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed manner. The software and data may be stored by one or more computer readable recording media.

The method according to the embodiments may be recorded in nontransitory computer-readable recording media including program instructions to implement various operations embodied by a computer. The computer-readable recording media may also include, alone or in combination with the program commands, data files, data structures, etc. The media and program instructions may be those specially designed and constructed for the purposes, or they may be of the kind well-known and available to those of skilled in the computer software arts. Examples of computer-readable media include magnetic media such as hard disks, floppy disks, and magnetic tape; optical media such as CD ROM disks and DVD; magneto-optical media such as floptical disks; and hardware devices that are specially to store and perform program commands, such as read-only memory (ROM), random access memory (RAM), flash memory, etc. Examples of the program commands may include not only machine language codes but also high-level language codes which are executable by various computing means by using an interpreter. The aforementioned hardware devices may be configured to operate as one or more software modules to carry out exemplary embodiments, and vice versa.

While the present disclosure has been described with reference to exemplary embodiments, one of ordinary skill in the art may practice various changes and modifications without departing from the spirit and scope of the present disclosure set forth throughout the annexed claim matters. For example, there may be attained a desired result according to the present disclosure even though the techniques are carried out through other methods and procedures different from the aforementioned, and/or even though the system, the structure, the units and the circuits are coupled in other manners different from the aforementioned, or substituted or replaced with other components or equivalents.

Therefore, other implementations, other embodiments, and equivalents to the claims are also within the scope of the following claims.

## Claims

1. A method of controlling liquid medication injection, the method comprising:
receiving, by an electronic device for controlling liquid medication injection, blood glucose values measured by a liquid medication injection device in which a liquid medication injection logic is programmed;
generating, by the electronic device for controlling liquid medication injection, a blood glucose change pattern by using the blood glucose values over time;
generating, by the electronic device for controlling liquid medication injection, a signal for changing the liquid medication injection logic taking into account a directivity of the change in the blood glucose value according to the blood glucose change pattern; and
transmitting, by the electronic device for controlling liquid medication injection, the signal for changing the liquid medication injection logic to the liquid medication injection device.

2. The method of claim 1, wherein
the generating of the signal for changing the liquid medication injection logic comprises
generating, by the electronic device for controlling liquid medication injection, the signal for changing the liquid medication injection logic taking into account a future blood glucose value according to the blood glucose change pattern and a directivity of the change.

3. The method of claim 1, wherein
the signal for changing the liquid medication injection logic is
one of a liquid medication injection stop signal or a liquid medication injection decrease signal.

4. The method of claim 1, further comprising,
after the transmitting to the liquid medication injection device,
calculating a current blood glucose value or a future blood glucose value based on a current time point, and determining whether to restart the liquid medication injection logic taking into account the current blood glucose value or the future blood glucose value, by the electronic device for controlling liquid medication injection.

5. The method of claim 1, further comprising,
after the transmitting to the liquid medication injection device,
when a preset first standby time expires, transmitting, by the electronic device for controlling liquid medication injection, a liquid medication injection logic restart signal or a liquid medication injection logic stop signal to the liquid medication injection device after re-determining whether to restart the liquid medication injection logic taking into account a directivity of the change in the blood glucose value according to the blood glucose change pattern that is calculated based on a current time point.

6. The method of claim 1, further comprising,
after the transmitting to the liquid medication injection device,
when a preset second standby time expires, automatically transmitting, by the electronic device for controlling liquid medication injection, a liquid medication injection logic restart signal to the liquid medication injection device.

7. The method of claim 1, further comprising,
after the transmitting to the liquid medication injection device,
receiving, by the electronic device for controlling liquid medication injection, a blood glucose value measured during a set injection stop time period from a time point when a liquid medication injection stop signal is generated;
determining, by the electronic device for controlling liquid medication injection, whether the received measured blood glucose value exceeds a normal blood glucose range; and
when the measured blood glucose value exceeds the normal blood glucose range, transmitting a signal for restarting the liquid medication injection logic.

8. A computer program stored in a computer-readable recording medium for executing one of the methods according to claims 1 to 5 by using a computer.

9. An electronic device for controlling liquid medication injection, which communicates with a liquid medication injection device in which a liquid medication injection logic is programmed, the electronic device comprising
a processor and a communication unit,
wherein the processor is configured to execute instructions included in an injection function controller, and
the injection function controller
receives the measured blood glucose values,
generates a blood glucose change pattern by using the blood glucose values over time,
generates a signal for changing the liquid medication injection logic taking into account the directivity of change in the blood glucose value according to the blood glucose change pattern, and
transmits the signal for changing the liquid medication injection logic to the liquid medication injection device.
